# EUROPEAN PATENT APPLICATION

(11) **EP 2 567 655 A2**
(43) Date of publication of application: **13.03.2013**
(21) Application number: 11777556.9
(22) Date of filing: 03.05.2011
(51) Int. Cl.: A61B 5/02, A61B 5/042, A61B 5/0428

(54) **SENSOR WHICH IS ATTACHABLE TO THE BODY, AND MONITORING APPARATUS**

(30) Priority: 03.05.2010 KR 20100041201
(71) Applicant: Korea Advanced Institute Of Science And Technology, Daejeon 305-701 (KR); Yoo, Hoe-Jun, Daejeon 305-701 (KR); Kim, Bin-Hee, Daejeon 305-701 (KR)
(72) Inventor: YOO, Hoi-jun, Daejeon 305-701 (KR); KIM, Bin-hee, Daejeon 305-701 (KR)
(74) Representative: Grünecker, Kinkeldey, Stockmair & Schwanhäusser
(86) International application number: PCT/KR2011/003312
(87) International publication number: WO 2011/139075

(57) **Abstract**

One embodiment of the present invention relates to a sensor for measuring biosignals. The sensor according to said embodiment comprises: a sensor layer formed by stacking a plurality of sensor layers that are attachable to the skin to measure different types of bio signals; a power for supplying power to the sensor layer; and a sensing electrode for sensing biosignals from the human body. The plurality of sensor layers takes the signals sensed by the sensing electrode as an input, and determines whether or not to measure the inputted signals. Then, the relevant sensor layer that can measure the sensed signal is activated.

## Description

### BACKGROUND OF THE INVENTION

### Field of the Invention

The present invention relates to a sensor attachable to the body and a monitoring apparatus including the sensor.

### Description of the Related Art

Recently, there has been proposed a monitoring apparatus configured in an adhesive plaster type or a para-aminosalicylic acid (PAS) type and to be attachable to the body, thereby enabling a patient to live without inconvenience in the state in which the patient wears the monitoring apparatus.

In this monitoring apparatus which is attachable to the body, a sensor can have a variety of sizes.

In general, if a sensor is an adhesive plaster type, the sensor is formed to have a size of 3 cm by 7 cm. If the sensor is a PAS type, the sensor is formed to have a size of 10 cm by 10 cm. In case where the monitoring apparatus is attached to the body, it is not desirable to blindly increase its area because of considering convenience of the user. In order to configure the sensor circuit within this limited area, it should be made by high-density structures. However, such the high-density circuit will increase the production cost of the apparatus.

### SUMMARY OF THE INVENTION

An object of an embodiment is to provide a sensor and a monitoring apparatus using such the sensor which are attachable to the body, wherein the sensor has a maximized area efficiency and give a wearable convenience to the user in the state in which the patient wears the monitoring apparatus.

A sensor according to an embodiment is an attachable sensor for measuring bio signals, including a sensor layer formed to be attachable to the skin and configured to have a plurality of sensor layers configured to measure different types of bio signals stacked thereon, a power source configured to supply power to the sensor layer, and sensing electrodes configured to sense the bio signals from the human body, wherein the plurality of sensor layers receives the respective signals sensed by the sensing electrodes and determines whether the received signals can be measured, and a corresponding sensor layer capable of measuring the sensed signal is activated.

The sensor preferably includes a flexible board, a circuit pattern installed on the flexible board, a plurality of passive elements connected to the circuit pattern, and a sensor chip connected to the circuit pattern and configured to measure the bio signals.

The flexible board preferably includes one of paper of pulp material, non-woven fabric, textiles, a patch of knitting, and a film.

The circuit pattern preferably is formed by forming a conductive paste on the flexible board in a circuit form using one of a silk screen method, a vacuum deposition method, and a sputtering deposition method or formed by cutting metal in a pattern of a circuit form and attaching the cut metal on the flexible board, and the sensor chip is bonded to the circuit pattern through flip-chip bonding, wire bonding, or tab bonding.

A sensor according to an embodiment is an attachable sensor for measuring bio signals, including a sensor layer configured to include a flexible board, a circuit pattern installed on the flexible board, a plurality of passive elements connected to the circuit pattern, and a sensor chip connected to the circuit pattern and configured to measure the bio signals, a power source configured to supply power to the sensor layer, and a sensing electrode configured to sense the bio signals from the human body, wherein a sensor circuit formed of the flexible board and the circuit pattern is plural, and the plurality of sensor circuits is coupled in a stack form.

The flexible board preferably includes one of paper of pulp material, non-woven fabric, textiles, a patch of knitting, and a film.

The circuit pattern preferably is formed by forming a conductive paste on the flexible board in a circuit form using one of a silk screen method, a vacuum deposition method, and a sputtering deposition method or formed by cutting metal in a pattern of a circuit form and attaching the cut metal on the flexible board, and the sensor chip is bonded to the circuit pattern through flip-chip bonding, wire bonding, or tab bonding.

A sensor according to an embodiment is an attachable sensor for measuring bio signals, including a sensor layer configured to include a flexible board, a circuit pattern installed on the flexible board, a plurality of passive elements connected to the circuit pattern, and a sensor chip connected to the circuit pattern and configured to measure the bio signals, a power source configured to supply power to the sensor layer, and a sensing electrode configured to sense the bio signals from the human body, wherein a sensor circuit formed of the flexible board and the circuit pattern is plural, and the plurality of sensor circuits is coupled in a stack form, a plurality of the sensor layers is configured so that the sensor layers measure different types of bio signals, respectively, and the plurality of sensor layers is stacked, and the plurality of sensor layers receives the respective signals sensed by the sensing electrodes and determines whether the received signals can be measured, and a corresponding sensor layer capable of measuring the sensed signal is activated.

The flexible board preferably includes one of paper of pulp material, non-woven fabric, textiles, a patch of knitting, and a film.

The circuit pattern preferably is formed by forming a conductive paste on the flexible board in a circuit form using one of a silk screen method, a vacuum deposition method, and a sputtering deposition method or formed by cutting metal in a pattern of a circuit form and attaching the cut metal on the flexible board, and the sensor chip preferably is bonded to the circuit pattern through flip-chip bonding, wire bonding, or tab bonding.

A monitoring apparatus according to an embodiment is a monitoring apparatus for monitoring bio signals, including a sensor of an adhesive plaster type formed to be attachable to the skin and configured to include a sensor layer configured to have a plurality of sensor layers for measuring different types of bio signals, respectively, stacked thereon, a power source configured to supply power to the sensor layer, and sensing electrodes configured to sense the bio signals from the human body, and an external terminal configured to receive measured data through the sensor and process the received data, wherein the plurality of sensor layers receives the respective signals sensed by the sensing electrodes and determines whether the received signals can be measured, and a corresponding sensor layer capable of measuring the sensed signal is activated.

The sensor preferably includes a flexible board, a circuit pattern installed on the flexible board, a plurality of passive elements connected to the circuit pattern, and a sensor chip connected to the circuit pattern and configured to measure the bio signals.

The flexible board preferably includes one of paper of pulp material, non-woven fabric, textiles, a patch of knitting, and a film.

The circuit pattern preferably is formed by forming a conductive paste on the flexible board in a circuit form using one of a silk screen method, a vacuum deposition method, and a sputtering deposition method or formed by cutting metal in a pattern of a circuit form and attaching the cut metal on the flexible board, and the sensor chip preferably is bonded to the circuit pattern through flip-chip bonding, wire bonding, or tab bonding.

The sensor chip preferably transmits the measured data to the external terminal wirelessly.

The monitoring apparatus preferably further includes an inductor connected to the sensor chip, wherein the inductor is connected to the external terminal using inductive coupling or radio frequency (RF) communication.

A monitoring apparatus according to an embodiment is a monitoring apparatus for monitoring bio signals, including a sensor of an adhesive plaster type configured to include a sensor layer comprising a flexible board, a circuit pattern installed on the flexible board, a plurality of passive elements connected to the circuit pattern, and a sensor chip connected to the circuit pattern and configured to measure the bio signals, a power source configured to supply power to the sensor layer, and a sensing electrode configured to sense the bio signals from the human body, and an external terminal configured to receive measured data through the sensor and process the received data, wherein a sensor circuit formed of the flexible board and the circuit pattern is plural, and the plurality of sensor circuits is coupled in a stack form.

The flexible board preferably includes one of paper of pulp material, non-woven fabric, textiles, a patch of knitting, and a film.

The circuit pattern preferably is formed by forming a conductive paste on the flexible board in a circuit form using one of a silk screen method, a vacuum deposition method, and a sputtering deposition method or formed by cutting metal in a pattern of a circuit form and attaching the cut metal on the flexible board, and the sensor chip preferably is bonded to the circuit pattern through flip-chip bonding, wire bonding, or tab bonding.

The sensor chip preferably transmits the measured data to the external terminal wirelessly.

The monitoring apparatus preferably further includes an inductor connected to the sensor chip, wherein the inductor is connected to the external terminal using inductive coupling or radio frequency (RF) communication.

A monitoring apparatus according to an embodiment is a monitoring apparatus for monitoring bio signals, including a sensor of an adhesive plaster type configured to include a sensor layer comprising a flexible board, a circuit pattern installed on the flexible board, a plurality of passive elements connected to the circuit pattern, and a sensor chip connected to the circuit pattern and configured to measure the bio signals, a power source configured to supply power to the sensor layer, and a sensing electrode configured to sense the bio signals from the human body, and an external terminal configured to receive measured data through the sensor and process the received data, wherein a sensor circuit formed of the flexible board and the circuit pattern is plural, and the plurality of sensor circuits is coupled in a stack form, a plurality of the sensor layers is configured so that the sensor layers measure different types of bio signals, respectively, and the plurality of sensor layers is stacked, and the plurality of sensor layers receives the respective signals sensed by the sensing electrodes and determines whether the received signals can be measured, and a corresponding sensor layer capable of measuring the sensed signal is activated.

The flexible board preferably includes one of paper of pulp material, non-woven fabric, textiles, a patch of knitting, and a film.

The circuit pattern preferably is formed by forming a conductive paste on the flexible board in a circuit form using one of a silk screen method, a vacuum deposition method, and a sputtering deposition method or formed by cutting metal in a pattern of a circuit form and attaching the cut metal on the flexible board, and the sensor chip preferably is bonded to the circuit pattern through flip-chip bonding, wire bonding, or tab bonding.

The sensor chip preferably transmits the measured data to the external terminal wirelessly.

The monitoring apparatus preferably further includes an inductor connected to the sensor chip, wherein the inductor is connected to the external terminal using inductive coupling or radio frequency (RF) communication.

According to the present invention, there is provided a wearable sensor and a monitoring with a maximized area efficiency.

### BRIEF DESCRIPTION OF THE DRAWINGS

FIG. 1A is a diagram showing an example of the construction of a monitoring apparatus in accordance with a first embodiment;
FIG. 1B is a diagram showing an example of the construction of a sensor layer in accordance with an embodiment;
FIG. 1C is a diagram showing the construction of the bottom of a sensor in accordance with an embodiment; and
FIG. 2 is a diagram showing an example of the construction of a monitoring apparatus in accordance with a second embodiment.

### DETAILED DESCRIPTION OF PREFERRED EMBODIMENTS

Reference will now be made in greater detail to a preferred embodiment of the invention, an example of which is illustrated in the accompanying drawings. Wherever possible, the same reference numerals will be used throughout the drawings and the description to refer to the same or like parts.

### [First embodiment]

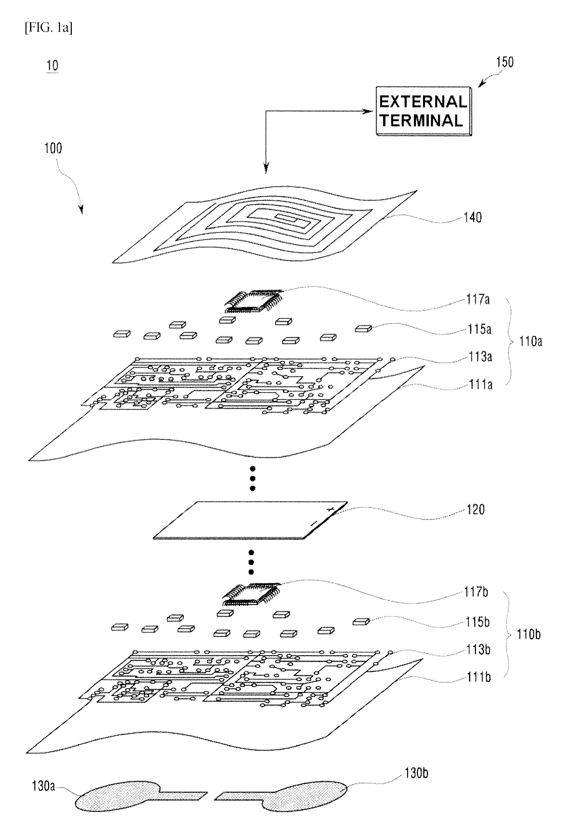

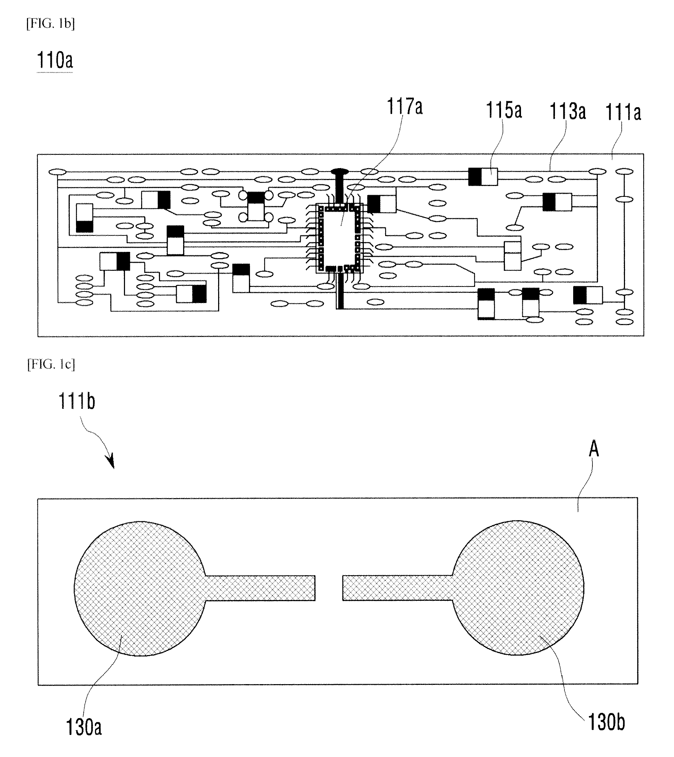

FIG. 1A is a diagram showing an example of the construction of a monitoring apparatus 10 in accordance with a first embodiment. FIG. 1B is a diagram showing an example of the construction of a first sensor layer 110a in accordance with an embodiment. FIG. 1C is a diagram showing the construction of the bottom of a sensor 100 in accordance with an embodiment.

Referring to FIGS. 1A to 1C, the monitoring apparatus 10 in accordance with the first embodiment includes a sensor 100 and an external terminal 150.

### Construction of the sensor 100

The sensor 100 includes a plurality of sensor layers 110a and 110b, a power source 120, and sensing electrodes 130a and 130b.

Each of the sensor layers 110a and 110b can be fabricated in an adhesive plaster type which can be attached to the skin. A plurality of the sensor layers 110a and 110b can be stacked. The plurality of sensor layers 110a and 110b can be configured to measure different types of bio signals, respectively. In the first embodiment, an example in which two sensor layers 110a and 110b (hereinafter referred to as a first sensor layer 110a and a second sensor layer 110b) are configured is described.

The first sensor layer 110a includes a flexible board 111a, a circuit pattern 113a, a plurality of passive elements 115a, and a sensor chip 117a.

The flexible board 111a can be configured to include one of paper of pulp material, non-woven fabric, textiles, a patch of knitting, and a film. Here, one of both faces of the flexible board 111a can be an adhesive surface having an adhesive property.

The circuit pattern 113a can be formed by forming a conductive paste on the flexible board 111a in a circuit form using one of a silk screen method, a vacuum deposition method, and a sputtering deposition method. Or, the circuit pattern 113a can be formed by cutting metal in a pattern of a circuit form and attaching the cut metal on the flexible board 111a. The circuit pattern 113a is formed on an opposite side to the adhesive surface of the flexible board 111a.

The passive elements 115a are installed on the circuit pattern 113a and can be electrically connected to the circuit pattern 113a.

The sensor chip 117a is installed on the circuit pattern 113a and can be electrically connected to the circuit pattern 113a. The sensor chip 117a can be connected to the circuit pattern 113a by flip-chip bonding, wire bonding, or tab bonding. The sensor chip 117a can be a bare die IC chip.

The sensor chip 117a can measure bio signals from the human body using the sensing electrodes 130a and 130b. The sensor chip 117a can amplify the measured bio signals, perform filtering processing on the amplified signals, and convert them into digital data. Furthermore, the sensor chip 117a can perform processing processes, such as compression/encryption, on the converted data, store the processed data, and transmit the stored data to the external terminal 150 wirelessly. Or, the sensor chip 117a can wirelessly transmit the measured raw data to the external terminal 150 without change.

Meanwhile, in case where the sensor chip 117a cannot be connected to the external terminal 150, an inductor 140(used as a RF antenna) can be additionally configured in the sensor layers 110a and 110b. The inductor 140 can be installed at the top of the sensor layers 110a and 110b, and it enables the sensor chip 117a and the external terminal 150 to be wirelessly coupled using inductive coupling circuit or Radio Frequency (RF) communication, functioning as a wireless module. Accordingly, the sensor chip 117a can transmit the measured data to the external terminal 150 through the inductor 140.

The sensor chip 117a can be configured to directly transmit the measured data wirelessly or can be configured to access the external terminal 150 through the inductor 140, but the present invention is not necessarily limited to the above constructions. The sensor chip 117a can be configured to transmit the data, stored in the sensor chip 117a, to the external terminal 150 through a wired method.

The second sensor layer 110b includes a flexible board 111b, a circuit pattern 113b, a plurality of passive elements 115b, and a sensor chip 117b. The second sensor layer 110b has a similar construction as the first sensor layer 110a. Here, the sensor chip 117a configured in the first sensor layer 110a and the sensor chip 117b configured in the second sensor layer 110b can be configured to measure different bio signals.

The first sensor layer 110a and/or the second sensor layer 110b can include one or more sensor circuits. Here, the sensor circuits mean circuits formed of the flexible boards 111a and 111b and the circuit patterns 113a and 113b. For example, if one sensor circuit is further configured in the first sensor layer 110a, the sensor circuit can be stacked under a sensor circuit that is basically configured in the first sensor layer 110a. Here, the added sensor circuit can be connected to the sensor circuit that is basically configured in the first sensor layer 110a, and the two stacked sensor circuits can operate as one sensor circuit. The two stacked sensor circuits do not need to necessarily come in contact with each other physically and another element layer can be stacked in a middle layer between the two stacked sensor layers. However, the two sensor circuits need to be electrically coupled. The construction of the sensor circuits stacked as described above can increase area efficiency because the limited space (or area) of the sensor is utilized to a maximum extent.

The power source 120 can be installed between the first sensor layer 110a and the second sensor layer 110b, between the sensor circuit and the sensor circuit, or at the top of the sensor layers 110a and 110b. The power source 120 can be connected to the sensor chips 117a and 117b, and it can supply power to the sensor layers 110a and 110b. The power source 120 can use a flexible battery or work based on wireless power transmission such as RFID.

FIG. 1C is a diagram showing the construction of the bottom of the sensor in accordance with an embodiment.

The sensing electrodes 130a and 130b are configured to sense bio signals from the human body and connected to the respective sensor chips 117a and 117b.

The sensing electrodes 130a and 130b can be installed between the first sensor layer 110a and the second sensor layer 110b or at the bottom of the sensor layers 110a and 110b. Here, the bottom of the sensor layers 110a and 110b can be the bottom of the flexible board 111b that is configured in the second sensor layer 110b.

The sensing electrodes 130a and 130b preferably are installed at the bottom of the sensor layers 110a and 110b in order to reduce a feeling of foreign material for the sensor 100. The second sensor layer 110b can be disposed at the bottom of the sensor layer. In this case, the sensing electrodes 130a and 130b can be installed on the adhesive surface A of the flexible board 111b that is configured in the second sensor layer 110b.

### Construction of the external terminal 150

The external terminal 150 can receive measured data through the sensor 100 and process the measured data. The external terminal 150 can perform processing processes, such as decompression/decoding, on the received data, and store the processed data.

The external terminal 150 can be a portable terminal the user is having so that the external terminal 150 remotely transmits information through a wire or wireless network and receives signals to control the sensor 100.

Furthermore, if the inductor 140 for performing communication using inductive coupling or RF communication is configured in the sensor 100, the external terminal 150 can be configured to perform communication with the sensor 100 using inductive coupling or RF communication.

Meanwhile, the data communication between the external terminal 150 and the sensor 100 can be performed by a wired manner or by a human body communication technology.

### Construction of the monitoring apparatus 100

First, when the sensor 100 is powered on, the sensor chips 117a and 117b can be initialized and connected to the power source 120.

Next, bio signals from the human body are sensed through the sensing electrodes 130a and 130b, and then the sensed bio signals are inputted to the sensor chips 117a and 117b. Each of the sensor chips 117a and 117b measures the received bio signal and determines whether the measured bio signal can be processed. Furthermore, each of the sensor chips 117a and 117b is activated or deactivated according to a result of the determination.

For example, if it is determined that the bio signals sensed by the sensing electrodes 130a and 130b are bio signals that can be measured by the first sensor layer 110a, the first sensor layer 110a can be activated, whereas the second sensor layer 110b can be deactivated. Accordingly, the first sensor layer 110a measures the bio signals from the human body using the sensing electrodes 130a and 130b.

In contrast, if it is determined that the bio signals sensed by the sensing electrodes 130a and 130b are bio signals that can be measured by the second sensor layer 110b, the second sensor layer 110b can be activated, whereas the first sensor layer 110a can be deactivated. Accordingly, the second sensor layer 110b measures the bio signals from the human body using the sensing electrodes 130a and 130b.

Furthermore, a sensor layer to be activated can be selected in response to an external command, and when the selected sensor layer is activated, the sensor 100 may operate.

Next, measured bio signals can be amplified and filtered through corresponding sensor chips and then converted into digital data. Furthermore, the converted data can be subject to processing processes, such as compression/encryption, through the corresponding sensor chips, and then stored, and the stored data can be transmitted to the external terminal 150. Or, the measured raw data can be transmitted to the external terminal 150 without change.

Meanwhile, if the inductor 140 is configured in the sensor 100, data stored in a corresponding sensor chip can be transmitted to the external terminal 150 through the inductor 140.

### [Second embodiment]

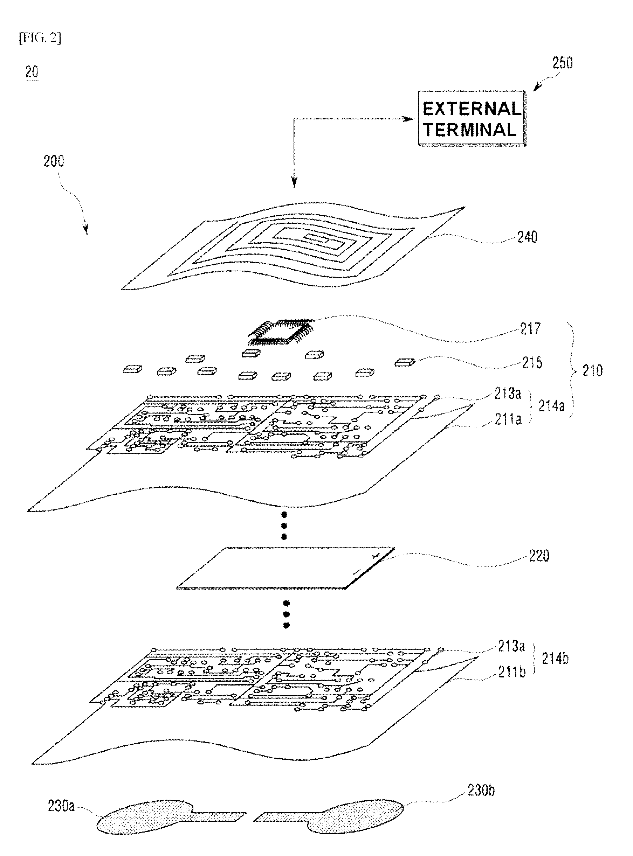

FIG. 2 is a diagram showing an example of the construction of a monitoring apparatus 20 in accordance with a second embodiment.

Referring to FIG. 2, the monitoring apparatus 20 in accordance with the second embodiment includes a sensor 200 and an external terminal 250. Being different the first embodiment, the second embodiment can reduce a surface area of the sensor, by providing the sensor 200 having a plurality of layers 210a and 210b.

### Construction of the sensor 200

The sensor 200 includes first and second sensor layers 210a and 210b, a power source 220, and sensing electrodes 230a and 230b.

The first sensor layer 210a can be fabricated in an adhesive plaster type which can be attached to the skin.

The first sensor layer 210a includes a plurality of passive elements 215, a sensor chip 217, a circuit pattern 213a, and a flexible board 211a. The second sensor layer 210a includes a flexible board 211b and a circuit pattern 213b. In FIG. 2, the second sensor layer 210b which does not include the sensor chip 217 can be configured by one array or a plurality of arrays.

The first and second sensor layers 210a and 210b can be stacked and electrically coupled. Here, the plurality of sensor layers can operate as one sensing circuit. The stacked construction as described above can increase area efficiency because the limited space (or area) of the sensor is utilized to a maximum extent.

The flexible boards 211a and 211b can be configured using paper of pulp material, non-woven fabric, textiles, a patch of knitting, and a film. Here, any one of both faces of the flexible boards 211a and 211b can be an adhesive surface having an adhesive property in order to be attached to human body for the sensor 200 or between sensor layers.

The circuit patterns 213a and 213b can be formed by forming a conductive paste on the respective flexible boards 211a and 211b in a circuit form using one of a silk screen method, a vacuum deposition method, and a sputtering deposition method. Or, the circuit patterns 213a and 213b can be formed by cutting metal in a pattern of a circuit form and attaching them to the flexible boards 211a and 211b, respectively.

Preferably, the circuit patterns 213a and 213b are formed on an opposite side to the adhesive surface of the flexible boards 211a and 211b.

The passive elements 215 are installed on the circuit pattern 213a and can be electrically connected to the circuit pattern 213a.

The sensor chip 217 is installed on the circuit pattern 213a and can be electrically connected to the circuit pattern 213a. The sensor chip 217 can be connected to the circuit pattern 213a through flip-chip bonding, wire bonding, or tab bonding. The sensor chip 217 can be a bare die IC chip.

The sensor chip 217 can measure bio signals from the human body using the sensing electrodes 230a and 230b. The sensor chip 217 can amplify and filter the measured bio signals and convert the processed data into digital data. Furthermore, the sensor chip 217 can perform processing processes, such as compression/encryption, on the converted data, store the processed data, and wirelessly transmit the stored data to the external terminal 250. Or, the sensor chip 217 can transmit the measured raw data to the external terminal 250 wirelessly without change.

Meanwhile, in case where the sensor chip 217 is configured so that it cannot be connected to the external terminal 250 in a wired manner, an inductor 240 can be additionally configured in the sensor layer 210. The inductor 240 can be installed at the top of the sensor layer 210, and it enables the sensor chip 217 and the external terminal 250 to be wirelessly coupled using inductive coupling or Radio Frequency (RF) communication. Accordingly, the sensor chip 217 can transmit information to the external terminal 250 through the inductor 240.

The sensor chip 217 can be configured to directly transmit the measured data wirelessly or can be configured to access the external terminal 250 through the inductor 240, but the present invention is not necessarily limited to the above constructions. The sensor chip 217 can be configured to transmit the data, stored in the sensor chip 217, to the external terminal 250 through a wired method.

In accordance with the second embodiment, the sensor layer 210a or 210b can be configured by one or more arrays. In this case, the one or more sensor layers 210 can be configured like the sensor layers 110a and 110b of the first embodiment, and the sensor 200 has the similar operating method as the sensor 100 of the first embodiment.

Furthermore, in case where it is necessary to integrate the circuits, each sensor layer can be configured to have a plurality of stacked structures.

The power source 220 can be installed between the stacked sensor circuits 214a and 214b or at the top of the sensor layer 210. The power source 220 can be connected to the sensor chip 217, and it can supply power to the sensor chip 217. The power source 220 can be a flexible battery.

The sensing electrodes 230a and 230b are configured to sense bio signals from the human body and are connected to the sensor chip 217.

The sensing electrodes 230a and 230b can be installed in the stacked sensor circuits 214a and 214b or at the bottom of the sensor circuits 214a and 214b. Here, the bottom of the sensor circuits 214a and 214b can be the bottom of the flexible board 211b that is configured at the lowest layer.

The sensing electrodes 230a and 230b preferably are installed at the bottom of the sensor layers 210a and 210b in order to reduce a feeling of foreign material for the sensor 200. In this case, the sensing electrodes 230a and 230b can be installed on the adhesive surface A of the flexible board 211b that is configured at the lowest layer.

### Construction of the external terminal 250

The external terminal 250 can receive measured data from the sensor 200 and process the received data. The external terminal 250 can perform processing processes, such as decompression/decoding, on the received data and store the processed data.

Furthermore, if the inductor 240 for performing communication using inductive coupling or RF communication is configured in the sensor 200, the external terminal 150 can be configured to communicate with the sensor 200 using inductive coupling or RF communication.

Furthermore, the external terminal 250 can be connected to the sensor 200 in a wired manner and configured to communication with the sensor 200.

In accordance with an embodiment, if one or more bio signals are measured or a space that forms circuits needs to be extended, sensors are configured by piling up the sensors in a multi-layer structure. Accordingly, the degree of integration of circuits can be increased because a limited area can be utilized to a maximum extent.

In accordance with an embodiment, the attachable sensor having maximized area efficiency and the monitoring apparatus including the sensor can be provided.

Although a preferred embodiment of the present invention has been described for illustrative purposes, those skilled in the art will appreciate that various modifications, additions and substitutions are possible, without departing from the scope and the spirit of the invention as disclosed in the accompanying claims.

The present invention is used in a sensor attachable to the body and a monitoring apparatus including the sensor.

## Claims

1. An attachable sensor for measuring bio signals, comprising:
a plurality of sensor layers formed to be attachable to a skin and configured to measure different types of bio signals;
a power source configured to supply power to the plurality of sensor layers; and
sensing electrodes configured to sense the bio signals from a human body,
wherein each of the plurality of sensor layers receives the bio signals through the sensing electrodes and measures the bio signals by activating the corresponding sensor layer in response to the received signals.

2. The attachable sensor according to claim 1, wherein any one of the plurality of sensor layers comprises:
a flexible board;
a circuit pattern installed on the flexible board;
a plurality of passive elements connected to the circuit pattern; and
a sensor chip connected to the circuit pattern and the sensing electrodes and configured to measure the bio signals.

3. The attachable sensor according to claim 1, wherein any one of the plurality of sensor layers comprises:
a first flexible board;
a first circuit pattern installed on the first flexible board;
a plurality of passive elements connected to the first circuit pattern; and
a sensor chip connected to the first circuit pattern and the sensing electrodes and configured to measure the bio signals,
wherein another sensor layer comprises:
a second flexible board; and
a second circuit pattern installed on the second flexible board.

4. The attachable sensor according to claim 2, wherein: the circuit pattern is formed by forming a conductive paste on the flexible board using one of a silk screen method, a vacuum deposition method, and a sputtering deposition method or formed by cutting metal in a pattern of a circuit form and attaching the cut metal on the flexible board, and
the sensor chip is bonded to the circuit pattern through flip-chip bonding, wire bonding, or tab bonding.

5. An attachable sensor for measuring bio signals, comprising:
a first sensor layer configured to comprise a first flexible board, a first circuit pattern installed on the first flexible board, a plurality of passive elements connected to the first circuit pattern, and a sensor chip connected to the circuit pattern and configured to measure the bio signals;
a power source configured to supply power to the first sensor layer;
a sensing electrode configured to sense the bio signals from a human body; and
one or more second sensor layers configured to receive power supply from the sensing electrode, being installed on the second flexible board, and comprising the second circuit pattern coupled to the sensor chip.

6. The attachable sensor according to claim 5, wherein the first and second flexible board comprises one of paper of pulp material, non-woven fabric, textiles, a patch of knitting, and a film.

7. The attachable sensor according to claim 5, wherein:
the first or second circuit pattern is formed by forming a conductive paste on the flexible board using one of a silk screen method, a vacuum deposition method, and a sputtering deposition method or formed by cutting metal in a pattern of a circuit form and attaching the cut metal on the flexible board; and
the sensor chip is bonded to the circuit pattern through flip-chip bonding, wire bonding, or tab bonding.

8. A sensor for measuring bio signals comprising:
a first sensor layer configured to comprise a first flexible board, a first circuit pattern installed on the first flexible board, a plurality of passive elements connected to the first circuit pattern, and a first sensor chip connected to the circuit pattern and configured to measure the bio signals;
a second sensor layer configured to comprise a second flexible board, a second circuit pattern installed on the second flexible board, a plurality of passive elements connected to the second circuit pattern, and a second sensor chip connected to the circuit pattern and configured to measure the bio signals;
a power source configured to supply power to the first and second sensor layers; and
a sensing electrode configured to sense the bio signals from a human body.

9. The sensor according to claim 8, wherein:
the first or second circuit pattern is formed by forming a conductive paste on the flexible board using one of a silk screen method, a vacuum deposition method, and a sputtering deposition method or formed by cutting metal in a pattern of a circuit form and attaching the cut metal on the flexible board; and
the sensor chip is bonded to the circuit pattern through flip-chip bonding, wire bonding, or tab bonding.

10. The apparatus for monitoring bio signals comprises:
a sensor, wherein the sensor includes a sensor layer formed to be attachable to a skin and configured to have a plurality of sensor layers configured to measure different types of bio signals, a power source configured to supply power to the plurality of sensor layers, and sensing electrodes configured to sense the bio signals from a human body and wherein the sensor is configured to receive signals sensed by the sensing electrodes and activate the sensor layers in response to the sensed input signal; and
an external terminal configured to receive and process signals measured by the sensor.

11. The apparatus according to claim 10, wherein any one of the plurality of sensor layers comprises:
a first flexible board;
a first circuit pattern installed on the first flexible board;
a plurality of passive elements connected to the first circuit pattern; and
a sensor chip connected to the first circuit pattern and the sensing electrodes and configured to measure the bio signals, and
wherein another sensor layer comprises:
a second flexible board; and
a second circuit pattern installed on the second flexible board.

12. The apparatus according to claim 10, wherein the flexible board comprises one of paper of pulp material, non-woven fabric, textiles, a patch of knitting, and a film.

13. The apparatus according to claim 10, wherein:
the circuit pattern is formed by forming a conductive paste on the flexible board using one of a silk screen method, a vacuum deposition method, and a sputtering deposition method or formed by cutting metal in a pattern of a circuit form and attaching the cut metal on the flexible board, and
the sensor chip is bonded to the circuit pattern through flip-chip bonding, wire bonding, or tab bonding.

14. The apparatus according to claim 10, wherein the sensor further includes an inductor connected to the sensor chip, wherein the inductor is connected to the external terminal using inductive coupling or radio frequency (RF) communication.

15. A apparatus for monitoring bio signals, comprising:
a first sensor layer configured to comprise a first flexible board, a first circuit pattern installed on the first flexible board, a plurality of passive elements connected to the first circuit pattern, and a sensor chip connected to the first circuit pattern and configured to measure the bio signals;
one or more second sensor layers configured to comprise a second flexible board and a second circuit pattern installed on the second flexible boar and connected to sensor chip;
a power source unit configured to supply power to the first and second sensor layers; and
a sensing electrode configured to sense the bio signals from a human body;
a sensor connected to the sensor chip and configured to comprise a wireless communication to perform wireless receiving/transmitting operations; and
a terminal configured to receive measured data through the sensor and process the received data through the wireless communication module.

16. The apparatus according to claim 15, wherein:
the first or second circuit pattern is formed by forming a conductive paste on the flexible board using one of a silk screen method, a vacuum deposition method, and a sputtering deposition method or formed by cutting metal in a pattern of a circuit form and attaching the cut metal on the flexible board; and
the sensor chip is bonded to the circuit pattern through flip-chip bonding, wire bonding, or tab bonding.
